# EUROPÄISCHE PATENTSCHRIFT

(11) **EP 3 636 348 B1**
(45) Veröffentlichungstag und Bekanntmachung des Hinweises auf die Patenterteilung: **04.01.2023**
(21) Anmeldenummer: 18199176.1
(22) Anmeldetag: 08.10.2018
(51) Int. Cl.: B05B 12/00, B05B 11/00

(54) **SET AUS EINEM PUMPSPENDER UND EINER AUSWERTEEINHEIT**
SET OF A PUMP DISPENSER AND AN EVALUATION UNIT
ENSEMBLE D'UN DISTRIBUTEUR DE POMPE ET D'UNE UNITÉ D'ÉVALUATION

(43) Veröffentlichungstag der Anmeldung: 15.04.2020
(73) Patentinhaber: Aptar Radolfzell GmbH, 78315 Radolfzell (DE)
(72) Erfinder: Jones, Matthew Meredith, Warwick, Warwickshire CV34 6AU (GB); Marsch, William Goeffrey Arthur, Buckingham, Buckinghamshire MK18 4HZ (GB); Graham, Dominic Alexander, Abingdon, Oxfordshire OX14 1XX (GB); Körner, Joachim, 88690 Uhldingen-Mühlhofen (DE)
(74) Vertreter: Witte, Weller und Partner Patentanwälte mbB Stuttgart

(56) Entgegenhaltungen:
- EP-A2- 2 177 141
- WO-A1-2005/080001
- US-A1- 2013 098 941
- US-A1- 2013 099 900

## Beschreibung

Die Erfindung betrifft ein Set aus einem Pumpspender zum Austrag von pharmazeutischen oder kosmetischen Flüssigkeiten und einer Auswerteeinheit.

Pumpspender im Sinne der Erfindung sind dabei die aus dem Stand der Technik grundsätzlich bekannten Pumpspender mit einem Flüssigkeitsspeicher, an dessen oberen Ende ein Austragkopf vorgesehen ist. Dieser Austragkopf verfügt über eine Basis, die am Flüssigkeitsspeicher befestigt ist, sowie über einen Betätigungsdrücker, der zum Zwecke des Austrags gegenüber der Basis niederdrückbar ist. Der Austragkopf verfügt über eine Pumpeinrichtung, die durch Niederdrücken des Betätigungsdrückers betätigt werden kann und die bei Betätigung Flüssigkeit von einem Einlasskanal, der mit dem Flüssigkeitsspeicher verbunden ist, zu einer am Austragkopf vorgesehenen Austragöffnung fördert.

Pumpspender der beschriebenen Art sind vergleichsweise einfache Vorrichtungen, die preisgünstig hergestellt werden können. Es besteht der Wunsch, die Nutzung solcher Pumpspender zu protokollieren, um Mehrwerte sowohl für den Nutzer als auch für den Hersteller von Pumpspendern bzw. den darin enthaltenen Flüssigkeiten zu schaffen. Allerdings sind die elektronischen Komponenten zur Erfassung und Weiterverarbeitung von Betätigungen des Pumpspenders vergleichsweise teuer, so dass die Integration in Pumpspender in der Regel nicht wirtschaftlich wäre.

Aus der US 2013/0098941 A1 ist ein Spender bekannt, der über eine Basis verfügt, der über Potentiometer oder anderweitige Sensoren die Kraft bei Betätigung des Spenders erfasst.

### AUFGABE UND LÖSUNG

Aufgabe der Erfindung ist es, einen Pumpspender der beschriebenen Art dahingehend weiterzubilden, dass ein Austrag erfasst wird, um datentechnisch weiterverarbeitet werden zu können.

Hierfür wird vorgeschlagen, ein Set aus einen Pumpspender der eingangs genannten Art und einer Auswerteeinheit zur Verfügung zu stellen, wobei die Auswerteeinheit zur Erfassung der Betätigung der Pumpeinrichtung ausgebildet ist.

Dabei ist der Grundgedanke der vorliegenden Erfindung, dass der Pumpspender um eine Auswerteeinheit ergänzt wird, die als externe Auswerteeinheit ausgebildet ist. Unter einer externen Auswerteeinheit im Sinne der Erfindung wird verstanden, dass diese für die Grundfunktionalität des Pumpspenders nicht erheblich ist. Der Pumpspender ist bei entfernter Auswerteeinheit separat funktionstauglich und kann in üblicher Weise verwendet werden. Die externe Auswerteeinheit des erfindungsgemäßen Sets ergänzt diesen Pumpspender und kann an der Außenseite des Pumpspenders derart angebracht werden, dass sie eine Betätigung durch Niederdrücken des Betätigungsdrückers erfassen kann. Sie ist in einfach lösbarer Weise am Pumpspender angebracht, so dass es dem Benutzer leicht fällt, den Pumpspender auszutauschen und die Auswerteeinheit mit einem neuen Pumpspender weiterzuverwenden.

Es ist ein im Weiteren noch näher erläuterter Erfassungssensor zur Erfassung der Betätigung des Pumpspenders durch Niederdrücken des Betätigungsdrückers vorgesehen. Dieser erzeugt ein digitales oder analoges elektrisches Signal, welches von weiteren elektronischen Komponenten der Auswerteeinheit weiterverarbeitet und/oder weitergeleitet werden kann. Der Erfassungssensor ist vorzugsweise als Weg- oder Kraft- bzw. Drucksensor ausgebildet.

Er ist bei an dem Pumpspender angebrachter Auswerteeinheit vorzugsweise im Kraftfluss angeordnet. Dieser Kraftfluss verläuft bei üblicher Betätigung vom den Betätigungsdrücker betätigenden Finger des Benutzers zum Betätigungsdrücker, von dort zur Basis des Austragkopfes und dem Flüssigkeitsspeicher bis zu der Hand des Benutzers, die bei üblicher Bedienung den Flüssigkeitsspeicher umgreift.

Der Erfassungssensor ist vorzugsweise derart angeordnet, dass er die Verlagerung zwischen Betätigungsdrücker und Basis des Austragkopfes erfasst oder dass er die Kraft oder den Druck zwischen dem Flüssigkeitsspeicher und der umfassenden Hand des Benutzers erfasst.

Bei einer erfindungsgemäßen Bauform ist die Auswerteeinheit als externe Auswerteeinheit ausgebildet, die über einen mindestens einseitig offenen Aufnahmebehälter verfügt, in den zumindest der Flüssigkeitsspeicher lösbar eingeschoben ist, so dass zumindest die Austragöffnung an einem ersten oberen Ende aus dem Aufnahmebehälter herausragt, wobei an einem gegenüberliegenden zweiten Ende des Aufnahmebehälters oder eines daran angebrachten Verschlusses der Erfassungssensor angeordnet ist, der beim Niederdrücken des Betätigungsdrückers durch den Flüssigkeitsspeicher kraftbeaufschlagt wird, so dass die Betätigung vom Erfassungssensor erfassbar ist.

Es handelt sich bei dem Erfassungssensor vorzugsweise um einen Drucksensor oder einen Kraftsensor, der die Kraft misst, mit der der Flüssigkeitsspeicher auf den Erfassungssensor drückt.

Grundsätzlich sind aber auch andere Formen von Sensoren als Erfassungssensor verwendbar. So kann beispielsweise ein Erschütterungs- oder Beschleunigungssensor als Erfassungssensor genutzt werden, der mittelbar die Betätigung erfasst, beispielsweise indem Erschütterungen sensiert werden, die durch das Vorbeifahren des Betätigungsdrückers an einer oder mehreren Rastkanten verursacht wird. Das Vorhandensein eines Erschütterungs- oder Beschleunigungssensors in der Auswerteeinheit ist darüber hinaus auch für andere Funktionen von Vorteil. So kann damit beispielsweise ermittelt werden, ob der Pumpspender vor Verwendung ausreichend geschüttelt worden ist.

Die Auswerteeinheit kann auch über mindestens einen Sensor verfügen, der zur Erfassung der im Flüssigkeitsspeicher verbliebenen Flüssigkeitsmenge vorgesehen ist. Je nach Ausgestaltung kann auch der genannte Drucksensor bei der erfindungsgemäßen Bauform im Ruhezustand des Sets erfassen, wie schwer der Pumpspender noch ist und hieraus auf die verbleibende Flüssigkeitsmenge rückschließen.

Darüber hinaus sind vielfältige weitere Sensoren denkbar, die zweckmäßig Teil der Auswerteeinheit sein können. So kann Sensorik vorgesehen sein, um zu erkennen, ob die Auswerteeinheit am Pumpspender angebracht ist. Auch ist es denkbar, dass ein für den Pumpspender eindeutiges Identifikationskennzeichen oder ein für die Flüssigkeit im Pumpspender spezifisches Identifikationskennzeichen vom Pumpspender ausgelesen werden, beispielsweise für RFID-Technologie. Weitere mögliche Sensoren sind Sensoren zur Erfassung der Flüssigkeitstemperatur und/oder der Umgebungstemperatur.

Die Auswerteeinheit kann derart ausgestaltet sein, dass die erfassten Daten unmittelbar in ihr gespeichert werden und/oder über eine Anzeigevorrichtung, insbesondere in Form von Leuchtdioden oder einem Display, dargestellt werden. Alternativ oder zusätzlich ist es bevorzugt, wenn die Auswerteeinheit über eine drahtlose Datenschnittstelle verfügt, insbesondere in Art einer Bluetooth-Schnittstelle, einer 4G-Schnittstelle oder einer 5G-Schnittstelle, über die erfasste Daten an einen Server oder ein separates Auswertegerät wie ein Mobiltelefon übermittelt werden.

Die Verwendung eines erfindungsgemäßen Sets gestattet es, an sich bekannte Pumpspender in einfacher Art und Weise derart zu ergänzen, dass die Handhabung durch den Benutzer erfassbar und weiterverarbeitbar ist. Hieraus ergeben sich verschiedene Nutzungsmöglichkeiten:
Aus Perspektive des Benutzers und insbesondere im Bereich kosmetischer Anwendungen ist die Möglichkeit der Datenauswertung von Vorteil, da der Benutzer seine Nutzungsart selbst kennenlernt und von einem auswertenden Programm Vorschläge hierzu erhalten kann. So kann ein solches Programm, welches insbesondere auf dem genannten externen Auswertegerät läuft, die Häufigkeit der Nutzung, die konkrete Art der Verwendung wie beispielsweise die Art des Drückens und ähnliches analysieren und Vorschläge für andere ergänzende Produkte wie insbesondere Kosmetikprodukte machen. Insbesondere wenn mehrere vom Benutzer verwendete Spender über Auswerteeinheiten der beschriebenen oder anderer Art verfügen, lassen sich zielgerichtet Vorschläge machen. Auch kann die Auswerteeinheit mittelbar oder unmittelbar den Füllstand eines Spenders prüfen und Nachbestellungen des entsprechenden Produktes automatisch oder halbautomatisch veranlassen.

Im Bereich pharmazeutischer Flüssigkeiten lässt sich die Einhalten von ärztlich vorgegebenen Anwendungszeiten kontrollieren. Die Daten können ggf. an den Arzt weitergegeben werden, so dass dieser einen Überblick über die Einhaltung der Zeiten erlangt.

Aus Perspektive des Herstellers der im Pumpspender gelagerter Flüssigkeit lassen sich wertvolle Daten zur typischen Nutzung ableiten. So kann erkannt werden, ob die Frequenz der Nutzung sich ändert oder wo die Verwendung des Pumpspenders üblicherweise stattfindet.

Der genannte Pumpspender ist ein im Wesentlichen üblicher Pumpspender. Es gibt keine zwingenden Anpassungen, die der Verwendung bekannter Pumpspender mit der beschriebenen Auswerteeinheit entgegenstehen. Dies erleichtert die Markteinführung des beschriebenen Systems. Nur wenn besondere Funktionalitäten gewünscht sind, wie beispielsweise die eindeutige Kennzeichnung eines Pumpspenders, insbesondere über ein RFID-Tag, bedarf es einer Anpassung des Pumpspenders.

Typische und für sich genommen bekannte Merkmale, die ein PumpspenderzurVerwendung in einem erfindungsgemäßen Set aufweist, sind die folgenden: Der Flüssigkeitsspeicher des Pumpspenders kann über eine Schnappverbindung oder eine Gewindeverbindung mit dem Austragkopf verbunden sein. Alternativ kann die Basis des Austragkopfes einstückig mit dem Flüssigkeitsspeicher ausgebildet sein. Der Flüssigkeitsspeicher ist vorzugsweise ein länglicher Körper, dessen Haupterstreckungsrichtung insbesondere vorzugsweise mit der Bewegungsrichtung des Betätigungsdrückers gegenüber der Basis übereinstimmt. Der Flüssigkeitsspeicher ist vorzugsweise im Wesentlichen kreiszylindrisch geformt, wobei er an einem oberen Ende üblicherweise mit einer Rastkante oder einem Gewinde zum Ankoppeln des Austragkopfes versehen ist. Der Flüssigkeitsspeicher ist im verkaufsfertigen Zustand mit einer Flüssigkeit befüllt, vorzugsweise mit einer kosmetischen oder einer pharmazeutischen Flüssigkeit, insbesondere mit einer Creme, einer Salbe oder einer Lotion.

Ein Pumpspender, der Teil eines erfindungsgemäßen Sets ist, kann an seiner Außenseite eine Beschriftung aufweisen, beispielsweise Markenzeichen, Hinweise auf Inhaltsstoffe der Flüssigkeit oder Verwendungshinweise.

Der Pumpspender weist gattungsgemäß eine Pumpeinrichtung auf, also eine Einrichtung, die beim Niederdrücken des Betätigungsdrückers eine Teilmenge der Flüssigkeit des Flüssigkeitsspeichers vom Flüssigkeitsspeicher isoliert und anschließend zur Austragöffnung leitet, wobei der für den Austrag vorgesehen Überdruck in der Pumpkammer vorzugsweise unmittelbar durch die Kraftbeaufschlagung des Betätigungsdrückers erzeugt wird. Insbesondere kann die Pumpeinrichtung eine Pumpkammer, sowie ein eingangsseitiges Einlassventil und ein ausgangsseitiges Auslassventil aufweisen, wobei das Einlassventil bei Unterdruck in der Pumpkammer gegenüber dem Flüssigkeitsspeicher öffnet und wobei das Auslassventil bei Überdruck in der Pumpkammer gegenüber einer umgebenden Atmosphäre öffnet. Die Pumpkammer ist volumetrisch veränderlich, wobei dies insbesondere vorzugsweise durch einen komprimierbaren Balg oder durch einen Pumpzylinder mit innenliegendem und beweglichem Pumpkolben erzielt wird.

Wenngleich Gestaltungen möglich sind, bei denen die Austragöffnung an der Basis des Austragkopfes vorgesehen ist, ist die bei Pumpspendern üblichere Gestaltung mit Austragöffnung am Betätigungsdrücker vorliegend bevorzugt. Bei einer solchen Gestaltung wird die Austragöffnung beim Niederdrücken des Betätigungsdrückers gegenüber der Basis des Austragkopfes ebenfalls niedergedrückt.

Bei der erfindungsgemäßen Bauform der Auswerteeinheit basiert das Funktionsprinzip darauf, dass der Benutzer mit seiner Hand nicht unmittelbar den Flüssigkeitsspeicher umgreift, sondern stattdessen den Aufnahmebehälter, auf dem sich der Flüssigkeitsspeicher bei Betätigung abstützt. Es kommt daher zwischen dem Flüssigkeitsspeicher einerseits und dem Aufnahmebehälter oder dessen Verschluss andererseits zu einer Kraftwirkung, die erfasst wird.

Bei einem erfindungsgemäßen Set weist die Auswerteeinheit vorzugsweise eine Sensoreinheit auf, die den Erfassungssensor umfasst. Diese Sensoreinheit ist zwischen dem Flüssigkeitsspeicher des Pumpspenders und einem Boden des Aufnahmebehälters oder einem bodenseitig angebrachten Verschluss angeordnet.

Die Anordnung des Erfassungssensors am unteren zweiten Ende des Aufnahmebehälters, gegebenenfalls im Bereich eines dort vorgesehenen Verschlusses, erlaubt eine sehr einfache Auswertung. Die Sensoreinheit wird bei Betätigung als Ganzes zusammengedrückt. Bei Erreichen eines mechanisch oder elektronisch vorgesehenen Schwellenwerts kann dann eine Betätigung von der Elektronik des Auswerteeinheit als stattgefunden wahrgenommen werden.

Insbesondere kann es sich bei dem so angeordneten Erfassungssensor um einen Kraftsensor oder Drucksensor handeln, der die Kraft oder den Druck misst, der bzw. die vom Flüssigkeitsspeicher in Richtung des Bodens des Aufnahmebehälters oder des dortigen Verschlusses wirkt.

Zwei Gestaltungsvarianten sind von der Erfindung umfasst:
Bei einem erfindungsgemäßen Set mit Auswerteeinheit kann bei einer ersten erfindungsgemäßen Gestaltungsvariante der Aufnahmebehälter am zweiten unteren Ende permanent geschlossen ausgebildet sein und an seinem ersten Ende eine Öffnung aufweisen, die ausreichend groß ist, damit zumindest der Flüssigkeitsspeicher eingeschoben werden kann. Unter permanent verschlossen wird auch eine Gestaltung verstanden, die zwar das Öffnen grundsätzlich zulässt, jedoch nicht in für den Benutzer akzeptablem Aufwand.

Bei dieser Gestaltungsvariante wird bestimmungsgemäß der Pumpspender von oben, also vom ersten Ende aus, in den Aufnahmebehälter eingeschoben. Der Aufnahmebehälter ist dabei ausreichend lang, dass zumindest der Flüssigkeitsspeicher vollständig oder nahezu vollständig (größer 80 %) im Aufnahmebehälter aufgenommen werden kann.

Damit der eingeschobene Pumpspender anschließend nicht aus dem Aufnahmebehälter herausfallen kann, ist bevorzugterweise der Aufnahmebehälter mit einer Halteeinrichtung versehen, durch die der zuvor eingeschobene Pumpspender kraftschlüssig oder formschlüssig gegen Herausgleiten gesichert werden kann. Eine solche Halteeinrichtung kann im einfachsten Falle eine elastisch auslenkbare Klinke sein. Wichtig ist dabei, dass diese beim gewollten Entfernen des Pumpspenders zum Zwecke des Austausches nicht zerstört zu werden braucht. Komplexere Systeme können auch vorsehen, dass die Halteeinrichtung ein vom Aufnahmebehälter separates Teil aufweist, welches nach dem Einschieben des Pumpspenders in den Aufnahmebehälter aufgesetzt wird und beispielsweise mittels eines Bajonetts oder eines Gewindes gesichert wird.

Bei einem erfindungsgemäßen Set mit Auswerteeinheit kann bei einer zweiten erfindungsgemäßen Gestaltungsvariante der Aufnahmebehälter beidseitig offen ausgebildet sein, wobei eine Öffnung am ersten Ende einen zumindest abschnittsweise kleineren lichten Querschnitt als ein Maximalquerschnitt des Pumpspenders aufweist, so dass der Pumpspender nur partiell, zumindest aber mit der Austragöffnung, von innen durch die Öffnung hindurchgeschoben werden kann. Bei dieser zweiten Gestaltungsvariante wird der Pumpspender bestimmungsgemäß vom gegenüberliegenden zweiten Ende, also von unten, eingeführt. Aufgrund der Größe der Öffnung des Aufnahmebehälters am oberen ersten Ende kann der Pumpspender dabei nur begrenzt weit eingeschoben werden.

Bei dieser zweiten Gestaltungsvariante ist vorzugsweise vorgesehen, dass die Auswerteeinheit einen Verschluss zum Verschließen des Aufnahmebehälters am zweiten Ende aufweist, wobei der Verschluss abnehmbar und ankoppelbar ausgestaltet ist, damit er gelöst werden kann, wenn der alte Pumpspender entfernt oder neu eingesetzt werden soll, und damit nach Einsetzen des neuen Pumpspenders dieser im Aufnahmebehälter gesichert werden kann.

Auch die Befestigung des Verschlusses am Aufnahmebehälter kann mittels eines Gewindes oder eines Bajonetts erfolgen. Auch eine reibschlüssige Kopplung ist grundsätzlich denkbar, wird aber nicht bevorzugt.

Der Verschluss kann als gegenüber dem Aufnahmebehälter deutlich kürzeres Teil ausgebildet sein, welches lediglich den Aufnahmebehälter am unteren Ende verschließt. Der Verschluss kann jedoch auch eine größere Länge aufweisen, so dass die Befestigung des Verschlusses am Aufnahmebehälter eher zentrisch zwischen den gegenüberliegenden Enden der Auswerteeinheit erfolgt.

Der Verschluss definiert gemeinsam mit dem Aufnahmebehälter einen Innenraum für den Pumpspender. Dieser Innenraum ist vorzugsweise ausreichend groß, damit mindestens der Flüssigkeitsspeicher des Pumpspenders vollständig oder nahezu vollständig (größer 80 %) darin aufgenommen werden kann.

Bei einer Gestaltung der Auswerteeinheit mit einem Verschluss der beschriebenen Art ist es von Vorteil, wenn der Verschluss selbst die Sensoreinheit aufweist, wobei hier mehrere Varianten möglich sind. Der Verschluss kann unmittelbar das Gehäuse für elektronische Komponenten der Sensoreinheit bilden. Alternativ kann die Sensoreinheit ein eigenes Gehäuse aufweisen, welches fest oder lösbar im Verschluss vorgesehen ist.

Der Aufnahmebehälter ist vorzugsweise zumindest abschnittsweise aus transparentem Material, insbesondere aus transparentem Kunststoff gefertigt. Die transparente Gestaltung des Aufnahmebehälters, die dann als gegeben angesehen wird, wenn eine Beschriftung auf dem Pumpspender auch nach dessen Einfügung in den Aufnahmebehälter lesbar ist, gestattet eben jene Lesbarkeit der Beschriftung. Hierdurch lassen sich unterschiedliche Pumpspender auch dann sicher identifizieren, wenn sie jeweils in Aufnahmebehältern von Auswerteeinheiten eingesetzt sind.

Auch bei einer Auswerteeinheit gemäß der zweiten Gestaltungsvariante ist es von Vorteil, wenn eine Schutzkappe vorgesehen ist. Grundsätzlich ist es denkbar, dass diese Schutzkappe jene ist, die auch bei Verwendung des Pumpspenders ohne Auswerteeinheit am Austragkopf des Pumpspenders befestigt werden kann. Eine solche Gestaltung limitiert jedoch die Bauweise der Auswerteeinheit, so dass es als bevorzugt angesehen wird, wenn das Set eine Schutzkappe umfasst, die zur lösbaren Befestigung am ersten Ende des Aufnahmebehälters ausgebildet ist. Die beim Pumpspender gegebenenfalls mitgelieferte eigene Schutzkappe findet dann keine Verwendung mehr und wird durch die am Aufnahmebehälter befestigbare Schutzkappe ersetzt. Diese Schutzkappe zur Befestigung am Aufnahmebehälter kann in üblicher Art und Weise mittels einer form- oder kraftschlüssig wirkenden Kopplung am Aufnahmebehälter lösbar befestigt werden.

Wie eingangs bereits erläutert, kann die Auswertung der erfassten Daten unmittelbar an der Auswerteeinheit erfolgen, so dass diese keinerlei Schnittstellen braucht, um die Daten weiterzureichen. Bevorzugt ist jedoch eine Gestaltung, bei der es der Auswerteeinheit möglich ist, erfasste Daten an ein weiteres Gerät zu übersenden, weil dies tiefergehende Analysen und bessere visuelle Aufbereitung der erfassten Daten ermöglicht.

Grundsätzlich ist dabei denkbar, dass die Auswerteeinheit selbst mit dem Internet verbunden ist, beispielsweise über WLAN, 4G- oder 5G-Funknetzwerke. Sie kann dann ohne Zwischenschaltung eines weiteren aktiven Geräts des Benutzers die erfassten Daten an einen Zentralserver senden.

Bevorzugt ist es allerdings, wenn das Set mit Pumpspender und Auswerteeinheit zusätzlich ein mobiles Auswertegerät umfasst, insbesondere in Form eines Mobiltelefons des Benutzers, welches über eine drahtlose Schnittstelle, insbesondere die genannte Bluetooth-Schnittstelle, mit der Auswerteeinheit verbunden ist. Auf diesem mobilen Auswertegerät können Programme zur Auswertung der erfassten Daten laufen. Auch können weitere Sensoren des Auswertegeräts herangezogen werden, um die Daten der Auswerteeinheit zu ergänzen. Beispielsweise ist eine globale Lokalisierung, wie sie zum Beispiel über GPS möglich ist, mit einem vergleichsweise großen technischen Aufwand verbunden, so dass es von Vorteil sein kann, die Auswerteeinheit von einer entsprechenden technischen Gestaltung freizuhalten und stattdessen das mobile Auswertegerät, also insbesondere ein Mobiltelefon mit einem Programm (App), zur Erfassung der Position heranzuziehen. Allerdings kann es durchaus zweckmäßig sein, die Auswerteeinheit selbst mit einer Einrichtung zur globalen Lokalisierung zu versehen, da nicht immer gewährleistet sein muss, dass das mobile Auswertegerät am gleichen Ort wie die Auswerteeinheit ist.

Zu den Daten, die die Auswerteeinheit oder das mobile Auswertegerät speichern oder auswerten, können die unmittelbar die Betätigung betreffenden Daten wie Zeit und Ort gehören. Es können aber auch Charaktermerkmale der Betätigung, wie beispielsweise die Betätigungsgeschwindigkeit oder die Hublänge ausgewertet werden. Auch die Auswertung von Umgebungsparametern, wie beispielsweise der Temperatur, kann zweckmäßig sein. Die Daten werden vorzugsweise entweder direkt von der Auswerteeinheit oder von dem mobilen Auswertegerät an einen Zentralserver versendet, der ergänzende Analysen, auch über mehrere Benutzer hinweg, ermöglicht.

Das Set umfasst vorzugsweise ein mobiles Auswertegerät, insbesondere in Form eines Mobiltelefons, welches über eine drahtlose Schnittstelle mit der Auswerteeinheit verbunden ist.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt einen üblichen Pumpspender, wie er aus dem Stand der Technik bekannt ist.
Die Fig. 2 und 3 zeigen zum Pumpspender der Fig. 1 in geschnittener Darstellung die Funktionsweise bei Betätigung.
Fig. 4 zeigt ein erfindungsgemäßes Set mit dem Pumpspender der Fig. 1 bis 3 sowie einer daran angebrachten Auswerteeinheit in einer ersten Gestaltungsvariante.
Fig. 5 und Fig. 6 zeigen das Set der Fig. 4 nach abgeschlossener Montage.
Die Fig. 7 zeigt zum Set der Fig. 4 in geschnittener Darstellung die Funktionsweise bei Betätigung.
Fig. 8 zeigt ein erfindungsgemäßes Set mit dem Pumpspender der Fig. 1 bis 3 sowie einer daran angebrachten Auswerteeinheit gemäß in einer zweiten Gestaltungsvariante.
Fig. 9 und Fig. 10 zeigen das Set der Fig. 8 nach abgeschlossener Montage.
Die Fig. 11 zeigt zum Set der Fig. 8 in geschnittener Darstellung die Funktionsweise bei Betätigung.

### DETAILLIERTE BESCHREIBUNG DERAUSFÜHRUNGSBEISPIELE

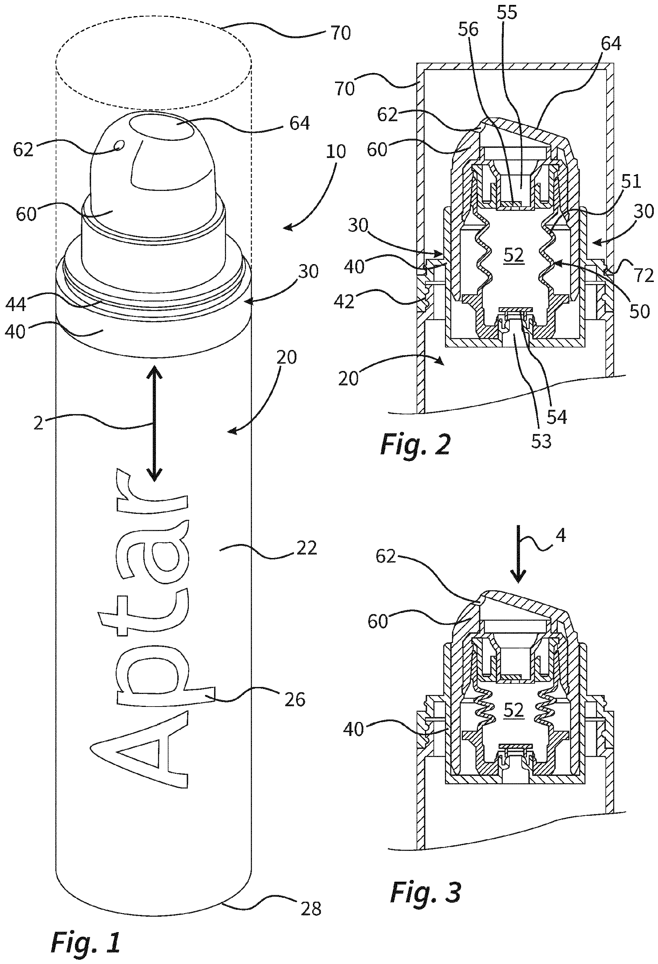

Die Fig. 1 bis 3 zeigen einen im Wesentlichen herkömmlichen Pumpspender, wie er Teil eines erfindungsgemäßen Sets wird.

Bezug nehmend auf Fig. 1 ist zu erkennen, dass der Pumpspender 10 über einen als nach oben offener Behälter ausgebildeten Flüssigkeitsspeicher 20 verfügt, auf dessen Mantelwandung 22 eine Beschriftung 26 vorgesehen ist. An den Flüssigkeitsspeicher ist mittels einer Kopplungseinrichtung 42 in Art eines Gewindes ein Austragkopf 30 angekoppelt. Dieser verfügt über einen in Richtung der Haupterstreckungsrichtung 2 des Pumpspenders niederdrückbaren Betätigungsdrücker 60, der bestimmungsgemäß manuell durch Kraftbeaufschlagung im Bereich einer Betätigungsfläche 64 niedergedrückt wird, um hierdurch Flüssigkeit aus dem Flüssigkeitsspeicher 20 durch eine am Betätigungsdrücker vorgesehene Austragöffnung 62 auszutragen. Im nicht benutzten Zustand kann der Austragkopf durch eine Schutzkappe 70 überdeckt werden, die im Bereich einer Rastkante 44 an der Basis 40 des Austragkopfs rastend befestigt ist.

Wie der Fig. 2 zu entnehmen ist, ist innerhalb des Austragkopfs 30 eine Pumpeinrichtung 50 vorgesehen. Diese umfasst eine durch Niederdrücken des Betätigungsdrückers 60 volumetrisch verkleinerbare Pumpkammer 52, die über einen Einlasskanal 53 mit dem Flüssigkeitsspeicher 20 und über einen Auslasskanal 55 mit der Austragöffnung 62 verbunden ist. Sowohl der Einlasskanal 53 als auch der Auslasskanal 55 verfügen jeweils über ein Ventil 54, 56. Das Einlassventil 54 öffnet bei Unterdruck in der Pumpkammer 52 gegenüber dem Flüssigkeitsspeicher 20 und somit bei einer nach oben gerichteten Rückstellbewegung des Betätigungsdrückers 60. In dieser Phase ist das Auslassventil 56 geschlossen, da es nur bei Überdruck in der Pumpkammer 52 gegenüber einer umgebenden Atmosphäre öffnet und anderenfalls schließt. Beim Niederdrücken des Betätigungsdrückers 60 hingegen öffnet sich das Auslassventil 56, während das Einlassventil 54 geschlossen ist. Auf diese Art und Weise ist in der in Fig. 3 ersichtlichen Weise möglich, Flüssigkeit aus der Pumpkammer 52 durch Niederdrücken des Betätigungsdrückers 60 in Betätigungsrichtung 4 zu bewirken. Beim vorliegenden Ausführungsbeispiel ist die Pumpeinrichtung 50 als Balgpumpeinrichtung ausgebildet und weist daher einen Balgkörper 51 auf, der die Pumpkammer 52 umgibt. Andere mögliche Bauweisen sehen beispielsweise eine Kolbenpumpe vor, bei der beim Niederdrücken des Betätigungsdrückers 60 ein Kolben innerhalb eines Zylinders zur Verkleinerung der Pumpkammer 52 verlagert wird.

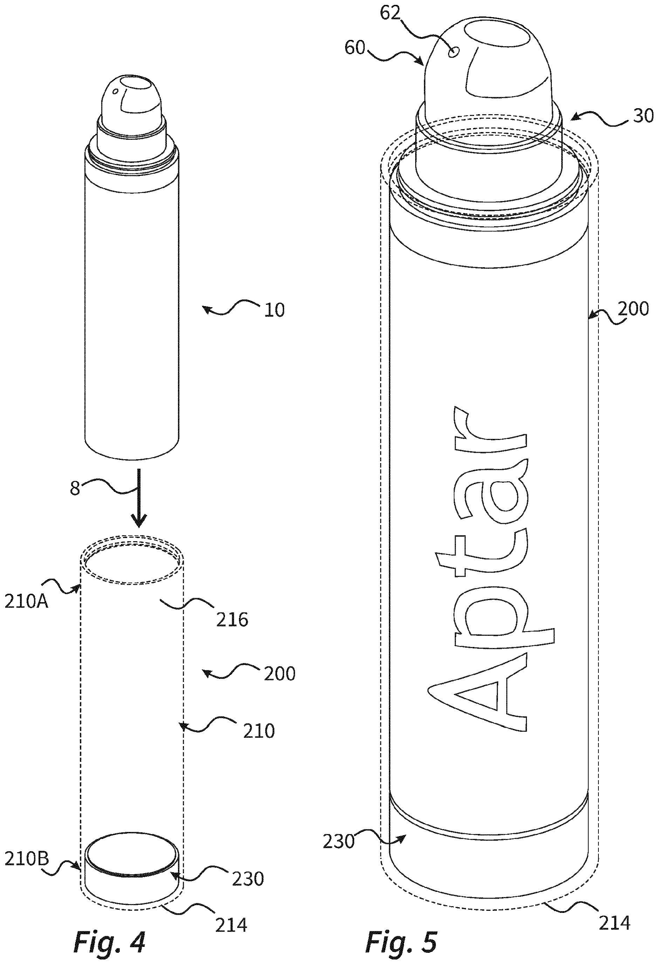

Die Fig. 4 bis 7 zeigen ein Ausführungsbeispiel der Erfindung. Hier findet ein Pumpspender 10 Verwendung, der derart ausgestaltet sein kann wie der in den Fig. 1 bis 3 dargestellte.

Zusätzlich umfasst ein Set entsprechend der Fig. 4 bis 7 eine Auswerteinheit 200, die primär durch einen Aufnahmebehälter 210 gebildet wird, auf dessen unterem Boden 214 eine Sensoreinheit 230 ruht. In dieser ist ein Kraft- oder Drucksensor 242 angeordnet, der durch Kraftbeaufschlagung an der Oberseite 232 der Sensoreinheit 230 ausgelöst werden kann. Der Aufnahmebehälter 210 weist eine Länge auf, durch die der Pumpspender 10 zum überwiegenden Teil und sein Flüssigkeitsspeicher 20 vollständig aufgenommen werden kann.

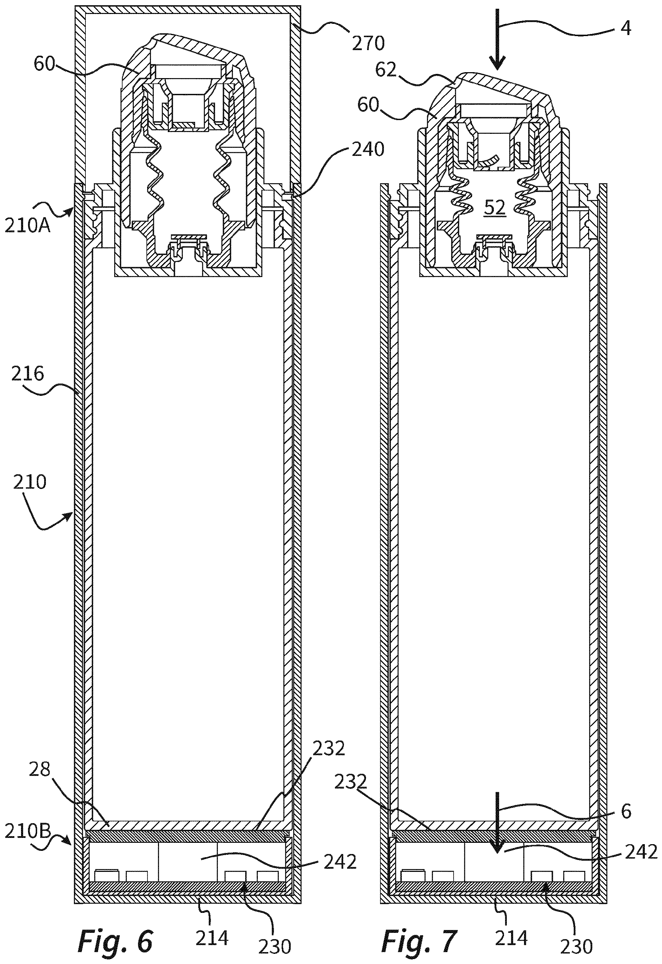

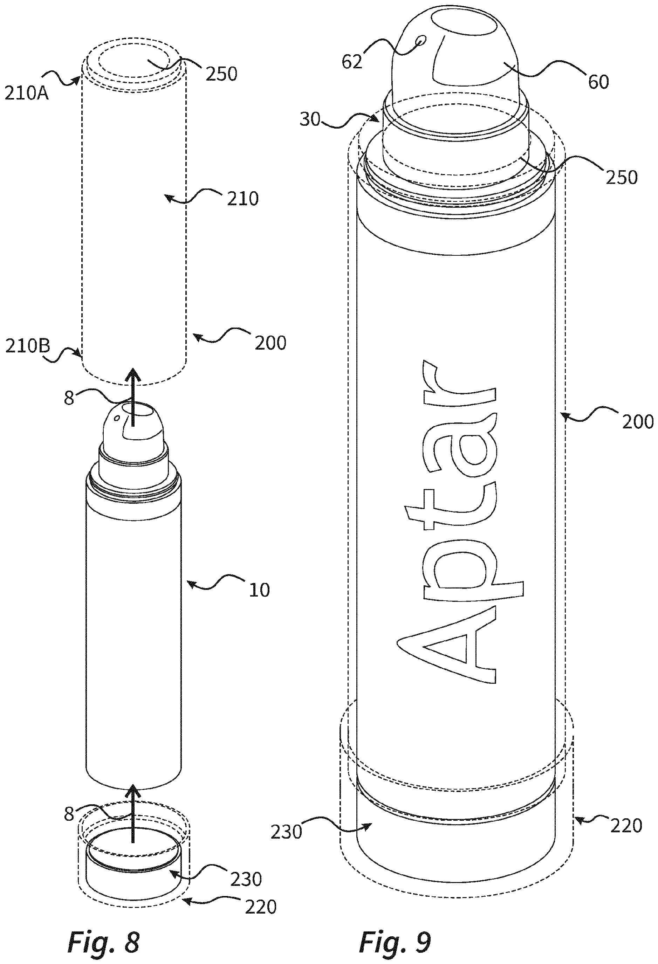

Fig. 6 zeigt den montierten Zustand. Es ist zu erkennen, dass eine Halteeinrichtung 240 in Form eines Rastrings an der Innenseite des Aufnahmebehälters 210 an dessen oberen Ende 210a dafür Sorge trägt, dass der eingefügte Pumpspender 10 nicht Gefahr läuft, aus dem Aufnahmebehälter 210 herauszurutschen. Weiterhin ist zu erkennen, dass der Flüssigkeitsspeicher 20 des Pumpspenders 10 mit seinem Boden 28 auf der Oberseite 232 der Sensoreinheit 230 aufliegt. Die Fig. 8 zeigt im Übrigen weiterhin, dass das Set über eine Schutzkappe 270 verfügt, die vorliegend über eine reibschlüssige Kopplung am oberen Ende 210a des Aufnahmebehälters 210 befestigt ist.

Fig. 7 verdeutlicht die Benutzung des Sets gemäß den Fig. 4 bis 6. Ein Benutzer umgreift während dieser Benutzung den Aufnahmebehälter 210 üblicherweise zumindest mit Daumen und Mittelfinger und betätigt den Betätigungsdrücker 60 in Betätigungsrichtung 4 mittels seines Zeigefingers. Die hierbei aufgebrachte Kraft bewirkt in der zuvor schon beschriebenen Art und Weise die volumetrische Verkleinerung der Pumpkammer 52 und somit den Austrag von Flüssigkeit aus der Pumpkammer 52.

Weiterhin wirkt diese Kraft jedoch, verdeutlicht durch den Pfeil 6, auch auf die Oberseite 232 der Sensoreinheit 230 und macht somit den Austragvorgang für die elektronischen Komponenten der Auswerteeinheit 200 erfassbar. Ausgehend von der erfassten Betätigung kann die Weiterverarbeitung in der oben beschriebenen Art erfolgen.

Die Fig. 8 bis 11 zeigen eine ähnlich geartete Gestaltung. Auch hier ist ein Pumpspender 10 entsprechend dem der Fig. 1 bis 3 vorgesehen, der in einem Aufnahmebehälter 210 aufgenommen wird.

In diesem Fall wird der Pumpspender 10 jedoch von unten in Montagerichtung in den Aufnahmebehälter 210 eingeschoben, so dass schlussendlich der Austragkopf 30 oder zumindest der Betätigungsdrücker 60 oben aus dem Aufnahmebehälter 210 hinausragt. Am unteren Ende 210B des Aufnahmebehälters wird ein Verschluss 220 in Montagerichtung 8 mittels eines Gewindes 222 befestigt, wobei dieser Verschluss eine Sensoreinheit 230 aufweist, die jener des vorangegangenen Ausführungsbeispiels ähnelt oder gleicht.

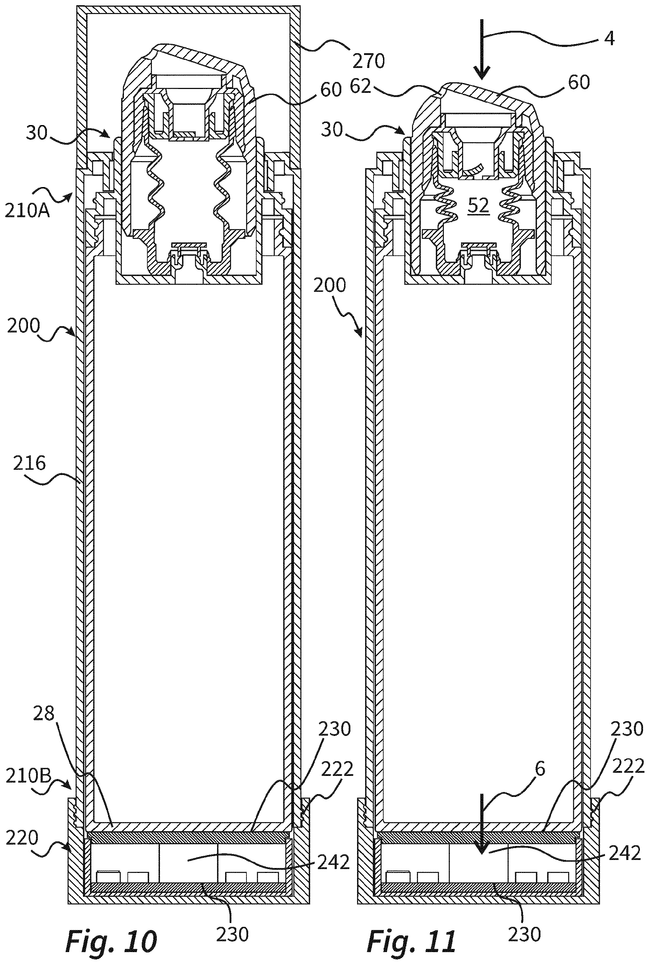

Die grundsätzliche Funktionsweise, dargestellt in den Fig. 10 und 11, ist die gleiche wie in den Fig. 6 und 7. Auch hier wirkt die Betätigung in Betätigungsrichtung 4 auf die Sensoreinheit 230, so dass der Kraft- oder Drucksensor 242 auslöst und eine Weiterverarbeitung dieser Information in der oben beschriebenen Weise ermöglicht.

Ein wesentlicher Unterschied ergibt sich nur beim Austausch des Pumpspenders. Bei der Gestaltung gemäß den Fig. 8 bis 11 ist zum Zwecke des Austausches zunächst der Verschluss 220 abzuschrauben, der Pumpspender 10 dann zu entfernen und durch einen neuen Pumpspender 10 auszutauschen, so dass dann der Verschluss 220 wieder aufgeschraubt werden kann.

Die beschriebenen Aufnahmebehälter 210 sind aus transparentem Kunststoff gefertigt, so dass die Beschriftung 26 trotz des Aufnahmebehälters lesbar bleibt.

Nicht dargestellt ist die Funktionsweise des Sets in Hinblick auf die Datenübermittlung. Der mit der Auswerteeinheit versehene Pumpspender 10 verfügt in seinem Aufnahmegehäuse über eine drahtlose Schnittstelle nach Bluetooth-Standard. Hierüber ist die Auswerteeinheit mit einem separaten Auswertegerät, vorliegend in der Art eines Mobiltelefons, gekoppelt. Dies bedeutet, dass die Auswerteeinheit bei jedem erfolgten Austrag oder bei Abwesenheit des Auswertegeräts zu einem späteren Zeitpunkt die Betätigung an das mobile Auswertegerät übermittelt, wo die Daten aggregiert und ausgewertet werden können. Auch können die Daten um weitere Daten ergänzt werden. So kann beispielsweise der GPS-Chipsatz des Mobiltelefons genutzt werden, um die Daten um den Ort der Betätigung zu ergänzen.

Das mobile Auswertegerät ist über das Internet mit einem Zentralserver verbunden, der insbesondere im Machtbereich des Herstellers des Pumpspenders stehen kann. Dieser Hersteller erhält dadurch Daten über die Benutzung, die er bei der Verbesserung des Produkts heranziehen kann. Auch kann der Zentralserver nach Auswertung der Daten vom mobilen Auswertegerät diesem Informationen zu Handlungsvorschlägen für die Benutzer schicken. Denkbar ist auch, dass der Füllstand des Spenders berücksichtigt wird und gegebenenfalls eine Nachlieferung eines Pumpspenders angeboten wird oder automatisch erfolgt.

Die Ermittlung des Füllstands des Pumpspenders ist einerseits indirekt darüber möglich, dass die Zahl der Austragvorgänge gezählt wird. Denkbar ist jedoch auch, dass beispielsweise die Sensoreinheit 230 in der Lage ist, anhand der im Ruhezustand auf sie einwirkenden Gewichtskraft des Pumpspenders 10 zu ermitteln, wie viel Flüssigkeit in diesem noch verblieben ist.

## Patentansprüche

1. Set aus einem Pumpspender (10) zum Austrag von pharmazeutischen oder kosmetischen Flüssigkeiten und einerAuswerteeinheit (200) den folgenden Merkmalen:
a. der Pumpspender (10) verfügt über einen Flüssigkeitsspeicher (20), an dessen oberen Ende ein Austragkopf (30) angebracht ist, und
b. der Austragkopf (30) verfügt über eine Basis (40), die am Flüssigkeitsspeicher (20) befestigt ist, sowie über einen Betätigungsdrücker (60), der zum Zwecke des Austrags gegenüber der Basis (40) niederdrückbar ist, und
c. der Austragkopf (30) verfügt über eine Pumpeinrichtung (50), die durch Niederdrücken des Betätigungsdrückers (60) betätigt werden kann und die bei Betätigung Flüssigkeit von einem Einlasskanal (53), der mit dem Flüssigkeitsspeicher (20) verbunden ist, zu einer am Austragkopf (30) vorgesehenen Austragöffnung (62) fördert, und
d. die Auswerteeinheit (200) ist zur Erfassung der Betätigung der Pumpeinrichtung (50) ausgebildet, wobei die Auswerteeinheit (200) als externe Auswerteeinheit (200) ausgebildet ist, die über einen mindestens einseitig offenen Aufnahmebehälter (210) verfügt, in den zumindest der Flüssigkeitsspeicher (20) lösbar eingeschoben ist, so dass zumindest die Austragöffnung (62) an einem ersten oberen Ende (210A) aus dem Aufnahmebehälter (210) herausragt, wobei an einem gegenüberliegenden zweiten Ende (210B) des Aufnahmebehälters (210) oder eines daran angebrachten Verschlusses (220) ein Erfassungssensor (242) angeordnet ist, der beim Niederdrücken des Betätigungsdrückers (60) durch den Flüssigkeitsspeicher (20) kraftbeaufschlagt wird, so dass die Betätigung vom Erfassungssensor (242) erfassbar ist,
**gekennzeichnet durch** eines der folgenden Merkmale:
e. der Aufnahmebehälter (210) weist eine Länge auf, die ausreichend groß ist, damit der Flüssigkeitsspeicher (20) vollständig oder nahezu vollständig darin aufgenommen sein kann, wobei der Aufnahmebehälter (210) am zweiten Ende permanent geschlossen ausgebildet ist und an seinem ersten Ende eine Öffnung aufweist, die ausreichend groß ist, damit zumindest der Flüssigkeitsspeicher (20) eingeschoben werden kann, oder
f. der Aufnahmebehälter (210) ist beidseitig offen ausgebildet, wobei eine Öffnung (250) am ersten Ende einen zumindest abschnittsweise kleineren lichten Querschnitt als ein Maximalquerschnitt des Pumpspenders (10) aufweist, so dass der Pumpspender (10) nur partiell, zumindest aber mit der Austragöffnung (62), von innen durch die Öffnung (250) hindurchgeschoben werden kann.

2. Set nach Anspruch 1 bei erfülltem Merkmal 1e mit den folgenden zusätzlichen Merkmalen:
a. die Auswerteeinheit (200) weist eine Sensoreinheit (230) auf, die den Erfassungssensor (242) umfasst, und
b. die Sensoreinheit (230) ist zwischen dem Flüssigkeitsspeicher (20) des Pumpspenders (10) und einem Boden (214) oder einem bodenseitig angebrachten Verschluss (220) angeordnet, vorzugsweise mit dem folgenden zusätzlichen Merkmal:
c. der Erfassungssensor (242) ist als Kraftsensor oder Drucksensor ausgebildet, der die Kraft oder den Druck misst, der bzw. die vom Flüssigkeitsspeicher (20) in Richtung des Bodens (214) des Aufnahmebehälters (210) oder des bodenseitigen Verschlusses (220) wirkt.

3. Set nach Anspruch 1 oder 2 bei erfülltem Merkmal 1e mit dem folgenden zusätzlichen Merkmal:
a. der Aufnahmebehälter (210) weist eine Halteeinrichtung (240) am ersten Ende auf, durch die der eingeschobene Pumpspender (10) kraftschlüssig oder formschlüssig gegen Herausgleiten gesichert werden kann.

4. Set nach Anspruch 1 bei erfülltem Merkmal 1f mit dem folgenden zusätzlichen Merkmal:
a. die Auswerteeinheit (200) weist einen Verschluss (220) zum Verschließen des Aufnahmebehälters am zweiten Ende (210B) auf, wobei der Verschluss (220) abnehmbar und ankoppelbar ausgestaltet ist,
vorzugsweise mit mindestens einem der folgenden weiteren Merkmale:
b. der Verschluss (220) begrenzt gemeinsam mit dem Aufnahmebehälter (210) einen Innenraum (260), der ausreichend groß ist, damit der Flüssigkeitsspeicher (20) vollständig oder nahezu vollständig darin aufgenommen sein kann, und/oder
c. der Verschluss (220) ist mittels eines Gewindes (222) oder einer Bajonettkupplung am Aufnahmebehälter ankoppelbar, und/oder
d. der Verschluss (220) weist die Sensoreinheit (230) auf, wobei der Verschluss (220) ein Gehäuse der Sensoreinheit bildet oder zur festen oder lösbaren Aufnahme eines Gehäuses der Sensoreinheit (230) ausgebildet ist.

5. Set nach einem der vorstehenden Ansprüche mit dem folgenden zusätzlichen Merkmal:
a. der Aufnahmebehälter (210) ist zumindest abschnittsweise aus transparentem Material, vorzugsweise aus transparentem Kunststoff gefertigt.

6. Set nach einem der vorstehenden Ansprüche mit dem folgenden zusätzlichen Merkmal:
a. DerAufnahmebehälter (210) weist eine Mantelwandung (216) auf, deren Formgebung im Wesentlichen der Formgebung einer Mantelwandung (22) des Flüssigkeitsspeichers (20) entspricht, wobei vorzugsweise die Mantelwandungen (22, 216) jeweils eine kreiszylindrische Form aufweisen.

7. Set nach einem der vorstehenden Ansprüche mit dem folgenden zusätzlichen Merkmal:
a. das Set umfasst eine Schutzkappe (270), die zur lösbaren Befestigung am ersten Ende (210A) des Aufnahmebehälters (210) ausgebildet ist.

8. Set nach einem der vorstehenden Ansprüche mit mindestens einem der folgenden zusätzlichen Merkmale:
a. der Flüssigkeitsspeicher (20) des Pumpspenders (10) ist über eine Schnappverbindung oder eine Gewindeverbindung mit dem Austragkopf (30) verbunden oder die Basis des Austragkopfes ist einstückig mit dem Flüssigkeitsspeicher ausgebildet, und/oder
b. der Flüssigkeitsspeicher (20) ist im Wesentlichen kreiszylindrisch geformt, und/oder
c. der Flüssigkeitsspeicher (20) ist im Bereich einer Mantelwandung (22) außenseitig mit einer Beschriftung (26) versehen, und/oder
d. die Pumpeinrichtung (50) weist eine Pumpkammer (52), ein eingangsseitiges Einlassventil (54) und ein ausgangsseitiges Auslassventil (56) auf, wobei das Einlassventil (54) bei Unterdruck in der Pumpkammer (52) gegenüber dem Flüssigkeitsspeicher (20) öffnet und wobei das Auslassventil (56) bei Überdruck in der Pumpkammer (52) gegenüber einer umgebenden Atmosphäre öffnet, und/oder
e. die Austragöffnung (62) ist am Betätigungsdrücker (60) angebracht, so dass sie beim Niederdrücken des Betätigungsdrückers (60) gegenüber der Basis (40) des Austragkopfes (30) ebenfalls niedergedrückt wird, und/oder
f. der Flüssigkeitsspeicher ist mit einer kosmetischen oder einer pharmazeutischen Flüssigkeit befüllt, insbesondere mit einer Creme, einer Salbe oder einer Lotion.

9. Set nach einem der vorstehenden Ansprüche mit dem folgenden zusätzlichen Merkmal:
a. das Set umfasst ein mobiles Auswertegerät (300), insbesondere in Form eines Mobiltelefons, welches über eine drahtlose Schnittstelle mit der Auswerteeinheit verbunden ist, vorzugsweise mit mindestens einem derfolgenden weiteren Merkmale:
b. die drahtlose Datenschnittstelle ist eine Bluetooth-Schnittstelle, und/oder
c. die Auswerteeinheit (200) verfügt über einen Speicher, in dem vom Erfassungssensor erfasste Daten abgelegt werden, um später bei bestehender Funkverbindung an das mobile Auswertegerät (300) oder einen Zentralserver (400) übertragen zu werden.

10. Set nach einem der vorstehenden Ansprüche mit mindestens einem der folgenden zusätzlichen Merkmale:
a. die Auswerteeinheit (200) verfügt über mindestens einen Sensor in Art eines Drucksensors (242), Kraftsensors (242) oder Wegsensors (140), insbesondere in Form eines Tasters (140), und/oder
b. die Auswerteeinheit (200) verfügt über mindestens einen Sensor (242), der zur Erfassung der im Flüssigkeitsspeicher verbliebenen Flüssigkeitsmenge vorgesehen ist, und/oder
c. die Auswerteeinheit (200) verfügt über mindestens einen Beschleunigungssensor, und/oder
d. die Auswerteeinheit (200) oder das mobile Auswertegerät (300) verfügt über mindestens
einen Sensor in Art einer globalen Positionserfassungseinrichtung, und/oder
e. die Auswerteeinheit (200) oder das mobile Auswertegerät (300) sind dafür ausgebildet, zu jeder durch den Erfassungssensor erfassten Betätigung die Zeit, den Ort, Charaktermerkmale der Betätigung oder Umgebungsparameter abzuspeichern und/oder über das Internet an einen Zentralserver zu versenden, und/oder
f. die Auswerteeinheit verfügt über eine Anzeigeeinrichtung, insbesondere in Form von Leuchtdioden oder einem Display (160).

## Claims

1. Set consisting of a pump dispenser (10) for dispensing pharmaceutical or cosmetic liquids and an evaluation unit (200) with the following features:
a. the pump dispenser (10) has a liquid store (20), to the upper end of which a dispensing head (30) is attached, and
b. the dispensing head (30) has a base (40) which is fastened to the liquid store (20), and a pushbutton (60) which can be pressed down with respect to the base (40) for the purpose of dispensing, and
c. the dispensing head (30) has a pumping device (50) which can be actuated by way of pressing down of the pushbutton (60), and which, in the case of actuation, conveys liquid from an inlet channel (53) which is connected to the liquid store (20) to a dispensing opening (62) which is provided on the dispensing head (30), and
d. the evaluation unit (200) is configured for the detection of the actuation of the pumping device (50), the evaluation unit (200) being configured as an external evaluation unit (200) which has a receiving container (210) which is open at least on one side and into which at least the liquid store (20) is inserted in releasably, with the result that at least the dispensing opening (62) protrudes out of the receiving container (210) at a first upper end (210A), a detection sensor (242) being arranged at an opposite second end (210B) of the receiving container (210) or of a closure (220) which is attached thereto, which detection sensor (242) is loaded with force by way of the liquid store (20) when the pushbutton (60) is pressed down, with the result that the actuation of the detection sensor (242) can be detected,
**characterized by** one of the following features:
e. the receiving container (210) has a length which is sufficiently great, in order that the liquid store (20) can be received completely or virtually completely therein, the receiving container (210) being of permanently closed configuration at the second end and, at its first end, having an opening which is sufficiently large, in order that at least the liquid store (20) can be pushed in, or
f. the receiving container (210) is of open configuration on both sides, an opening (250) at the first end having at least locally a smaller clear cross section than a maximum cross section of the pump dispenser (10), with the result that the pump dispenser (10) can be pushed from the inside through the opening (250) only partially, but at least with the dispensing opening (62).

2. Set according to Claim 1 in the case of feature 1e being met, with the following additional features:
a. the evaluation unit (200) has a sensor unit (230) which comprises the detection sensor (242), and
b. the sensor unit (230) is arranged between the liquid store (20) of the pump dispenser (10) and a bottom (214) or a closure (220) which is attached on the bottom side,
preferably with the following additional feature:
c. the detection sensor (242) is configured as a force sensor or pressure sensor which measures the force or the pressure which acts from the liquid store (20) in the direction of the bottom (214) of the receiving container (210) or of the bottom-side closure (220).

3. Set according to Claim 1 or 2 in the case of feature 1e being met, with the following additional feature:
a. the receiving container (210) has, at the first end, a holding device (240), by way of which the pushed-in pump dispenser (10) can be secured in a non-positive or positively locking manner against sliding out.

4. Set according to Claim 1 in the case of feature 1f being met, with the following additional feature:
a. the evaluation unit (200) has a closure (220) for closing the receiving container at the second end (210B), the closure (220) being configured such that it can be removed and coupled,
preferably with at least one of the following further features:
b. the closure (220) delimits, together with the receiving container (210), an interior space (260) which is sufficiently large, in order that the liquid store (20) can be received completely or virtually completely therein, and/or
c. the closure (220) can be coupled by means of a thread (222) or a bayonet coupling to the receiving container, and/or
d. the closure (220) has the sensor unit (230), the closure (220) forming a housing of the sensor unit or being configured to receive a housing of the sensor unit (230) fixedly or releasably.

5. Set according to one of the preceding claims with the following additional feature:
a. the receiving container (210) is manufactured at least in portions from transparent material, preferably from transparent plastic.

6. Set according to one of the preceding claims with the following additional feature:
a. the receiving container (210) has a shell wall (216), the shape of which corresponds substantially to the shape of a shell wall (22) of the liquid store (20), the shell walls (22, 216) preferably in each case having a circular-cylindrical shape.

7. Set according to one of the preceding claims with the following additional feature:
a. the set comprises a protective cap (270) which is configured to be fastened releasably to the first end (210A) of the receiving container (210).

8. Set according to one of the preceding claims with at least one of the following additional features:
a. the liquid store (20) of the pump dispenser (10) is connected via a snap-action connection or threaded connection to the dispensing head (30), or the base of the dispensing head is configured in one piece with the liquid store, and/or
b. the liquid store (20) is of substantially circular-cylindrical shape, and/or
c. the liquid store (20) is provided with an inscription (26) on the outer side in the region of the shell wall (22), and/or
d. the pumping device (50) has a pumping chamber (52), an inlet-side inlet valve (54) and an outlet-side outlet valve (56), the inlet valve (54) opening in the case of negative pressure in the pumping chamber (52) with respect to the liquid store (20), and the outlet valve (56) opening in the case of positive pressure in the pumping chamber (52) with respect to the surrounding atmosphere, and/or
e. the dispensing opening (62) is attached to the pushbutton (60), with the result that, in the case of the pushbutton (60) being pressed down with respect to the base (40) of the dispensing head (30), it is likewise pressed down, and/or
f. the liquid store is filled with a cosmetic or pharmaceutical liquid, in particular with a cream, an ointment or a lotion.

9. Set according to one of the preceding claims with the following additional feature:
a. the set comprises a mobile evaluation device (300), in particular in the form of a mobile telephone which is connected via a wireless interface to the evaluation unit,
preferably with at least one of the following further features:
b. the wireless data interface is a Bluetooth interface, and/or
c. the evaluation unit (200) has a memory, in which data which are detected by the detection sensor are stored, in order to be transmitted later to the mobile evaluation device (300) or a central server (400) when a radio connection is established.

10. Set according to one of the preceding claims with at least one of the following additional features:
a. the evaluation unit (200) has at least one sensor in the form of a pressure sensor (242), force sensor (242) or displacement sensor (140), in particular in the form of a switch (140), and/or
b. the evaluation unit (200) has at least one sensor (242) which is provided for the detection of the liquid quantity which remains in the liquid store, and/or
c. the evaluation unit (200) has at least one accelerometer, and/or
d. the evaluation unit (200) or the mobile evaluation device (300) has at least one sensor in the form of a global position detection device, and/or
e. the evaluation unit (200) or the mobile evaluation device (300) is configured to store the time, the location, characteristic features of the actuation or ambient parameters in the case of every actuation which is detected by way of the detection sensor, and/or to transmit them via the Internet to a central server, and/or
f. the evaluation unit has a display device, in particular in the form of light emitting diodes or a display (160).

## Revendications

1. Ensemble composé d'un distributeur à pompe (10) pour décharger des liquides pharmaceutiques ou cosmétiques et d'une unité d'évaluation (200), présentant les caractéristiques suivantes :
a. le distributeur à pompe (10) dispose d'un réservoir de liquide (20) à l'extrémité supérieure duquel est placée une tête de déchargement (30), et
b. la tête de déchargement (30) dispose d'une base (40) qui est fixée au réservoir de liquide (20), ainsi que d'un poussoir d'actionnement (60) qui peut être enfoncé par rapport à la base (40) dans le but du déchargement, et
c. la tête de déchargement (30) dispose d'un dispositif de pompage (50) qui peut être actionné par enfoncement du poussoir d'actionnement (60) et qui, lors de l'actionnement, achemine du liquide d'un canal d'admission (53), qui est relié au réservoir de liquide (20), vers une ouverture de déchargement (62) prévue sur la tête de déchargement (30), et
d. l'unité d'évaluation (200) est configurée pour détecter l'actionnement du dispositif de pompage (50), l'unité d'évaluation (200) étant configurée sous la forme d'une unité d'évaluation externe (200) qui dispose d'un réceptacle (210) ouvert au moins d'un côté, dans lequel au moins le réservoir de liquide (20) est inséré de manière amovible, de telle sorte qu'au moins l'ouverture de déchargement (62) fait saillie hors du réceptacle (210) à une première extrémité supérieure (210A), un capteur de détection (242) étant agencé à une deuxième extrémité opposée (210B) du réceptacle (210) ou d'une fermeture (220) qui y est placée, lequel est sollicité par force par le réservoir de liquide (20) lors de l'enfoncement du poussoir d'actionnement (60), de telle sorte que l'actionnement peut être détecté par le capteur de détection (242),
**caractérisé par** l'une des caractéristiques suivantes :
e. le réceptacle (210) présente une longueur qui est suffisamment grande pour que le réservoir de liquide (20) puisse y être entièrement ou presque entièrement logé, le réceptacle (210) étant configuré fermé en permanence à la deuxième extrémité et présentant à sa première extrémité une ouverture qui est suffisamment grande pour qu'au moins le réservoir de liquide (20) puisse être inséré, ou
f. le réceptacle (210) est configuré ouvert des deux côtés, une ouverture (250) à la première extrémité présentant une section transversale libre au moins par sections plus petite qu'une section transversale maximale du distributeur à pompe (10), de telle sorte que le distributeur à pompe (10) ne peut être poussé que partiellement, mais au moins par l'ouverture de déchargement (62), de l'intérieur à travers l'ouverture (250).

2. Ensemble selon la revendication 1, lorsque la caractéristique 1e est satisfaite, présentant les caractéristiques supplémentaires suivantes :
a. l'unité d'évaluation (200) présente une unité de capteur (230) qui comprend le capteur de détection (242), et
b. l'unité de détection (230) est agencée entre le réservoir de liquide (20) du distributeur à pompe (10) et un fond (214) ou une fermeture (220) disposée du côté du fond, de préférence présentant la caractéristique supplémentaire suivante :
c. le capteur de détection (242) est configuré sous forme de capteur de force ou de capteur de pression qui mesure la force ou la pression exercée par le réservoir de liquide (20) en direction du fond (214) du réceptacle (210) ou de la fermeture (220) du côté du fond.

3. Ensemble selon la revendication 1 ou 2, lorsque la caractéristique 1e est satisfaite, présentant la caractéristique supplémentaire suivante :
a. le réceptacle (210) présente un dispositif de retenue (240) à la première extrémité, par lequel le distributeur à pompe (10) inséré peut être bloqué par force ou par complémentarité de forme pour empêcher qu'il ne sorte en glissant.

4. Ensemble selon la revendication 1, lorsque la caractéristique 1f est satisfaite, présentant la caractéristique supplémentaire suivante :
a. l'unité d'évaluation (200) présente une fermeture (220) pour fermer le réceptacle à la deuxième extrémité (210B), la fermeture (220) étant conçue de manière à pouvoir être retirée et accouplée,
de préférence présentant au moins l'une des autres caractéristiques suivantes :
b. la fermeture (220) délimite conjointement avec le réceptacle (210) un espace intérieur (260) qui est suffisamment grand pour que le réservoir de liquide (20) puisse y être entièrement ou presque entièrement logé, et/ou
c. la fermeture (220) peut être accouplée au réceptacle au moyen d'un filetage (222) ou d'un accouplement à baïonnette, et/ou
d. la fermeture (220) présente l'unité de capteur (230), la fermeture (220) formant un boîtier de l'unité de capteur ou étant configurée pour recevoir de manière fixe ou amovible un boîtier de l'unité de capteur (230).

5. Ensemble selon l'une quelconque des revendications précédentes, présentant la caractéristique supplémentaire suivante :
a. le réceptacle (210) est fabriqué au moins par sections en matériau transparent, de préférence en matière plastique transparente.

6. Ensemble selon l'une quelconque des revendications précédentes, présentant la caractéristique supplémentaire suivante :
a. le réceptacle (210) présente une paroi d'enveloppe (216) dont la mise en forme correspond essentiellement à la mise en forme d'une paroi d'enveloppe (22) du réservoir de liquide (20), les parois d'enveloppe (22, 216) présentant de préférence chacune une forme cylindrique circulaire.

7. Ensemble selon l'une quelconque des revendications précédentes, présentant la caractéristique supplémentaire suivante :
a. l'ensemble comprend un capuchon de protection (270) qui est configuré pour être fixé de manière amovible à la première extrémité (210A) du réceptacle (210).

8. Ensemble selon l'une quelconque des revendications précédentes, présentant au moins l'une des caractéristiques supplémentaires suivantes :
a. le réservoir de liquide (20) du distributeur à pompe (10) est relié à la tête de déchargement (30) par l'intermédiaire d'une liaison par encliquetage ou d'une liaison filetée, ou la base de la tête de déchargement est configurée d'un seul tenant avec le réservoir de liquide, et/ou
b. le réservoir de liquide (20) a une forme essentiellement cylindrique circulaire, et/ou
c. le réservoir de liquide (20) est pourvu d'une inscription (26) sur le côté extérieur dans la zone d'une paroi d'enveloppe (22), et/ou
d. le dispositif de pompage (50) présente une chambre de pompage (52), une soupape d'admission (54) côté entrée et une soupape de sortie (56) côté sortie, la soupape d'admission (54) s'ouvrant en cas de dépression dans la chambre de pompage (52) par rapport au réservoir de liquide (20) et la soupape de sortie (56) s'ouvrant en cas de surpression dans la chambre de pompage (52) par rapport à une atmosphère environnante, et/ou
e. l'ouverture de déchargement (62) est placée sur le poussoir d'actionnement (60) de telle sorte qu'elle est également enfoncée lors de l'enfoncement du poussoir d'actionnement (60) par rapport à la base (40) de la tête de déchargement (30), et/ou
f. le réservoir de liquide est rempli d'un liquide cosmétique ou pharmaceutique, notamment d'une crème, d'une pommade ou d'une lotion.

9. Ensemble selon l'une quelconque des revendications précédentes, présentant la caractéristique supplémentaire suivante :
a. l'ensemble comprend un appareil d'évaluation mobile (300), notamment sous la forme d'un téléphone mobile, qui est relié à l'unité d'évaluation par l'intermédiaire d'une interface sans fil,
de préférence présentant au moins l'une des autres caractéristiques suivantes :
b. l'interface de données sans fil est une interface Bluetooth, et/ou
c. l'unité d'évaluation (200) dispose d'une mémoire dans laquelle des données détectées par le capteur de détection sont enregistrées afin d'être transmises ultérieurement à l'appareil d'évaluation mobile (300) ou à un serveur central (400) lorsque la liaison radio existe.

10. Ensemble selon l'une quelconque des revendications précédentes, présentant au moins l'une des caractéristiques supplémentaires suivantes :
a. l'unité d'évaluation (200) dispose d'au moins un capteur du type capteur de pression (242), capteur de force (242) ou capteur de déplacement (140), notamment sous la forme d'un palpeur (140), et/ou
b. l'unité d'évaluation (200) dispose d'au moins un capteur (242) qui est prévu pour détecter la quantité de liquide restant dans le réservoir de liquide, et/ou
c. l'unité d'évaluation (200) dispose d'au moins un capteur d'accélération, et/ou
d. l'unité d'évaluation (200) ou l'appareil d'évaluation mobile (300) dispose d'au moins un capteur du type dispositif de détection de position globale, et/ou
e. l'unité d'évaluation (200) ou l'appareil d'évaluation mobile (300) sont configurés pour mémoriser l'heure, le lieu, les caractéristiques de l'actionnement ou les paramètres environnementaux pour chaque actionnement détecté par le capteur de détection et/ou pour les envoyer à un serveur central par l'intermédiaire d'Internet, et/ou
f. l'unité d'évaluation dispose d'un dispositif d'affichage, notamment sous la forme de diodes électroluminescentes ou d'un écran (160).
